**Europäisches Patentamt**

**European Patent Office**

**Office européen des brevets**

(19)

(11) Veröffentlichungsnummer: **0 094 489**
**B1**

(12) # EUROPÄISCHE PATENTSCHRIFT

(45) Veröffentlichungstag der Patentschrift:
16.09.87

(51) Int. Cl.⁴: **A 61 B 17/58**

(21) Anmeldenummer: **83102694.3**

(22) Anmeldetag: **18.03.83**

(54) **Knochennagel zur Versorgung von Frakturen im proximalen Oberschenkelbereich und zugehöriges Instrumentarium.**

(30) Priorität: **18.05.82 DE 8214493 U**

(43) Veröffentlichungstag der Anmeldung:
**23.11.83 Patentblatt 83/47**

(45) Bekanntmachung des Hinweises auf die Patenterteilung:
**16.09.87 Patentblatt 87/38**

(84) Benannte Vertragsstaaten:
**AT CH DE FR GB IT LI**

(56) Entgegenhaltungen:
**DE - A - 2 527 460**
**DE - C - 824 239**
**GB - A - 1 428 653**
**US - A - 3 208 450**
**US - A - 4 055 172**

(73) Patentinhaber: **Howmedica International, Inc.**
**Zweigniederlassung Kiel,**
**Professor-Küntscher-Strasse 1-5, D-2301 Schönkirchen**
**üb. Kiel (DE)**

(72) Erfinder: **Harder, Hans Erich, Mecklenburger Strasse 35,**
**D-2301 Probsteierhagen (DE)**
Erfinder: **Krämer, Hermann, Dr., Fohlenweg 30,**
**D-2350 Neumünster (DE)**

(74) Vertreter: **Dipl.-Ing. H. Hauck Dipl.-Phys. W. Schmitz**
**Dipl.-Ing. E. Graalfs Dipl.-Ing. W. Wehnert Dr.-Ing. W.**
**Döring, Neuer Wall 41, D-2000 Hamburg 36 (DE)**

ACTORUM AG

**Beschreibung**

Die Erfindung bezieht sich auf einen dünnen flexiblen Femurnagel zur Versorgung von Frakturen des proximalen Femurs gemäss dem Oberbegriff des Anspruchs 1.

Ein derartiger Nagel ist aus der US-A-4 055 172 bekanntgeworden. Er wird normalerweise zusammen mit mehreren verwendet (Bündelnagelung). Die einzelnen aus Metall bestehenden Knochennägel haben distal einen Kupplungsabschnitt für den Eingriff mit einem Einschlagwerkzeug und sind zwischen dem distalen und proximalen Abschnitt bogenförmig gekrümmt. Sie sind ausreichend elastisch, damit sie über ein Fenster im Condyl des Femurs in den Knochenkanal des Femurs eingetrieben werden können. Das proximale Ende des Nagels sucht seinen Weg im Knochenkanal und gelangt über den Femurhals in den Femurkopf. Mit Hilfe des Eintreibwerkzeugs wird der Knochennagel unter Beobachtung auf einem Bildverstärker im Knochenkanal vorgetrieben, wobei insbesondere der erste Nagel zur Reposition des Knochenfragments mehr oder weniger gedreht werden muss. Der Kupplungsabschnitt wird von einer quer zur Krümmungsebene orientierten Abplattung am distalen Ende gebildet, die zum Eingriff mit einem Einschlagwerkzeug ausserdem mit einem Loch versehen sind. Loch und Abplattung haben verhältnismässig scharfe Kanten. Das proximale Ende des Nagels ist relativ spitz ausgebildet.

Aus der GB-A-1 428 653 ist ein Knochennagel zur Versorgung von Handgelenkfrakturen bekannt, wobei der Nagel in die Speiche eingetrieben wird und proximal eine Verdickung aufweist. Die Verdickung soll ein Durchbohren der Kortikalis beim Implantieren verhindern.

Der Erfindung liegt die Aufgabe zugrunde, einen Femurnagel zur Versorgung von proximalen Femurfrakturen zu schaffen, mit dem sichergestellt ist, dass ungewollte Knochenverletzungen im Bereich des proximalen Femurs vermieden werden.

Diese Aufgabe wird durch die Merkmale des Kennzeichnungsteils des Anspruchs 1 gelöst.

Der erfindungsgemässe Femurnagel weist am proximalen Ende eine wulstartige, abgerundete Verdickung auf, die ihrerseits am freien Ende eine Abflachung aufweist, deren Ebene zur Aufnahme der Belastung im Femurkopf im Winkel zur Achse des proximalen Nagelabschnitts verläuft. Die Verdickung verhindert ein Durchbohren der Kortikalis im Femurkopf beim Eintreiben der Nägel. Insgesamt dient jedoch die Verdickung mit der Abflachung dazu, die auf den Femurkopf wirkende Belastung auf den Nagel zu übertragen. Nägel der erfindungsgemässen Art werden normalerweise so eingeschlagen, dass sie die Kortikalis nicht berühren. Durch Sintern der Fraktur kann jedoch ein Absacken des proximalen Femurs erfolgen, so dass das proximale Ende sich gleichwohl gegen die Kortikalis anlegt. In beiden Fällen dient Abflachung dazu, eine möglichst geringe Flächenpressung zu erzeugen und damit eine hohe Kraftaufnahme für den Nagel zu ermöglichen, ohne dass die Knochenrinde durchbohrt wird.

Der eingangs erwähnte bekannte Knochennagel ist im Querschnitt kreisförmig. Für das von den Knochennägeln zu fordernde Widerstandsmoment ist jedoch weitgehend der Nageldurchmesser in der Krümmungsebene entscheidend. Dies bedeutet, dass senkrecht zur Krümmungsebene der Nagel reduziert werden kann, ohne dass dadurch das Widerstandsmoment wesentlich beeinflusst wird. Bei derartig geformten Nagelquerschnitten lässt sich eine nicht unbeträchtliche Verringerung des Nagelmaterials erzielen. Daher sieht eine Ausgestaltung der Erfindung vor, dass der Querschnitt des Nagels über seine Länge polygonal ist mit abgerundeten Kanten. Hierbei liegt die Hauptachse vorzugsweise in der Krümmungsebene. Der polygonale Querschnitt kann zum Beispiel von einem gleichseitigen Dreieck gebildet werden. Auch bei dieser Querschnittsform wird Material eingespart, ohne dass das Widerstandsmoment gegenüber einem Nagel mit vergleichbarem Kreisquerschnitt merklich reduziert wäre. Da die Kanten des dreieckförmigen Querschnitts abgerundet sind, besteht auch keine Verletzungsgefahr im Knochenkanal. Ferner ist ohne weiteres gewährleistet, dass der Nagel zu Repositions- und Eintreibzwecken gedreht werden kann. Ein dreieckförmiger Querschnitt hat jedoch den weiteren Vorteil, dass eine engere Packung möglich ist als bei einem Kreisquerschnitt, so dass ggf. mehr Nägel eingetrieben werden können als im Querschnitt kreisförmige Knochennägel. Das Gesamtwiderstandsmoment ist bei gegebenem Querschnitt des Knochenkanals höher einstellbar als bei Rundnägeln.

Alternativ kann auch ein rechteckförmiger Querschnitt des Knochennagels vorgesehen werden, wobei die grössere Achse in der Krümmungsebene liegt. Bei einer derartigen Querschnittsform wird immer noch eine Materialeinsparung erzielt.

Eine platzsparende Anordnung der distalen Nagelenden ergibt sich dann, wenn die radiale Erstreckung des distalen Endes nicht über die des übrigen Nagels hinausgeht, d.h. die Aussenkontur des Nagels sich im distalen Endbereich nicht vergrössert. In diesem Zusammenhang sieht eine weitere Ausgestaltung der Erfindung vor, dass am distalen Ende eine Bohrung vorgesehen ist, vorzugsweise eine Durchbohrung, deren Achse vorzugsweise in der Krümmungsebene liegt. Ein Abschnitt des Eintreibwerkzeugs kann mit der Bohrung in Eingriff gebracht werden, damit auf das distale Nagelende eine Torsion aufgebracht werden kann.

Alternativ kann am distalen Nagelende eine Ausnehmung geformt sein zur Bildung eines im Querschnitt polygonalen Einspannabschnitts. Bei einem polygonalen Querschnitt des Nagels wäre eine Ausnehmung im Hinblick auf das Aufbringen einer Drehkraft nicht notwendig. Eine Ausnehmung hat jedoch den Vorteil, dass dadurch auch wirksame Auszugskräfte auf den Nagel aufgebracht werden können.

In weiteren Unteransprüchen sind weitere vorteilhafte Ausgestaltungen der Erfindung angegeben.

Ausführungsbeispiele der Erfindung werden nachfolgend anhand von Zeichnungen näher erläutert.

Fig. 1 zeigt einen Schnitt durch einen Femur, in den drei erfindungsgemässe Knochennägel implantiert sind.

Fig. 2 zeigt eine Seitenansicht des distalen Endes eines Knochennagels nach Fig. 1.

Fig. 3 zeigt einen Schnitt durch den Knochennagel nach Fig. 2 entlang der Linie 3–3.

Fig. 4 zeigt ein anderes Profil eines erfindungsgemässen Knochennagels.

Fig. 5 zeigt ein weiteres Profil eines erfindungsgemässen Knochennagels.

Fig. 6 zeigt ein Ausführungsbeispiel eines proximalen Endes des Knochennagels nach Fig. 1.

Fig. 7 zeigt ein Vorschlaggerät für einen erfindungsgemässen Knochennagel.

Fig. 8 zeigt einen Schnitt durch den vorderen Abschnitt des Geräts nach Fig. 7.

Fig. 9 zeigt ein Nachschlaggerät für einen erfindungsgemässen Knochennagel.

Fig. 10 zeigt eine um 90° gedrehte Ansicht des hinteren Endes des Geräts nach Fig. 9.

Fig. 11 zeigt eine Vorderansicht des Geräts nach Fig. 9.

Fig. 12 zeigt einen Schnitt durch das Gerät nach Fig. 9 entlang der Linie 12–12.

Fig. 13 zeigt einen Querschnitt durch eine andere Ausführungsform eines Nagels.

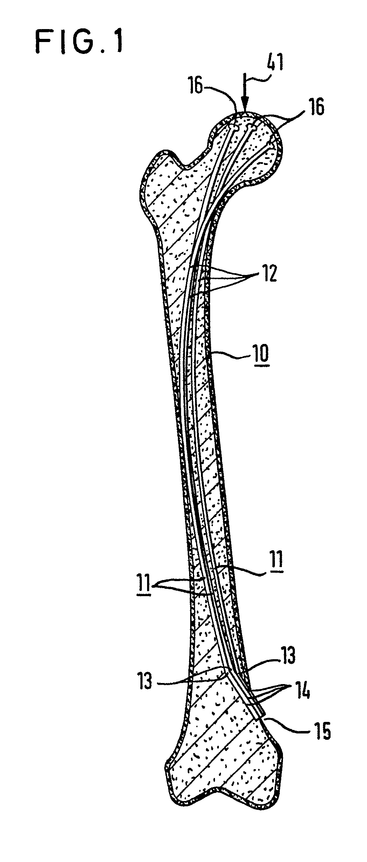

Die drei in Fig. 1 in einem Femur 10 implantierten Knochennägel sind allgemein mit 11 bezeichnet. Sie sind, vom proximalen Endabschnitt ausgehend mit einer durchgehenden Krümmung versehen, wie bei 12 dargestellt. Im Bereich des distalen Endes ist eine Abknickung 13 geformt, der ein gerades Stück 14 folgt. Die Knochennägel 11 sind über ein Fenster 15 des medialen Kondylus des gezeigten Femurs 10 eingetrieben mit Hilfe eines nicht dargestellten Einschlagwerkzeugs, und zwar ein Knochennagel nach dem anderen. Der erste eingetriebene Nagel dient normalerweise zur Reponierung des Fraktursegmentes. Das Einführen der Knochennägel erfolgt unter Kontrolle mit Hilfe eines Bildverstärkers, so dass sich im Kopfbereich des Femurs eine Auffächerung erzielen lässt, wodurch eine gewisse Drehstabilität des Kopfes relativ zum übrigen Femur erzielt wird. Die proximalen Endabschnitte sind mit Verdickungen versehen, die in Fig. 1 allgemein mit 16 bezeichnet sind.

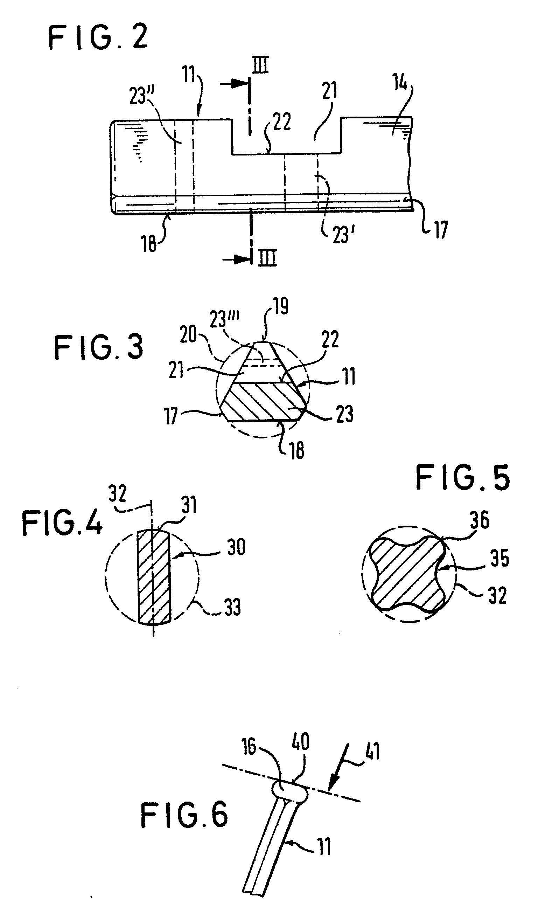

Wie aus Fig. 3 zu ersehen, ist der Querschnitt der Nägel 12 ein gleichseitiges Dreieck, dessen Eckkanten, wie bei 17 gezeigt, stark gerundet sind. Eine Seite 18 bildet dabei die konvexe Seite der Krümmung 12, während die gegenüberliegende Kante 19 auf der konkaven Seite des Nagels 11 liegt.

Wie durch den gestrichelt gezeichneten Kreis 20 angedeutet, hat der Nagel 11 gegenüber einem Nagel mit dem Kreisquerschnitt 20 eine erhebliche Materialeinsparung zur Folge, ohne dass das Widerstandsmoment in der Krümmungsebene wesentlich geringer wäre. Die in Fig. 3 dargestellte Querschnittsform hat ferner den Vorteil, dass sich die Nägel im Knochenkanal besser «packen» lassen.

Wie ferner aus den Figuren 2 und 3 hervorgeht, ist in den geraden distalen Endabschnitt 14 eine Ausnehmung 21 geformt, deren Boden 22 annähernd parallel zur Seite 18 des Nagels 11 verläuft. Mit der Ausnehmung 21 kann ein Einschlagwerkzeug in Eingriff gebracht werden, um den Nagel im Knochenkanal vorzutreiben oder ihn aus dem Knochenkanal zu extrahieren und bei diesen Bewegungen zusätzlich eine Drehbewegung auszuführen. Der verbleibende Restquerschnitt 23 reicht ohne weiteres aus, um Drehkräften standzuhalten. Es empfiehlt sich dabei, den Übergang des Querschnitts 23 auf den dreieckförmigen Vollquerschnitt stetig zu wählen, um unnötig hohe Kerbkräfte zu vermeiden.

Man erkennt aus den Figuren 2 und 3, dass trotz eines Kupplungsabschnittes für ein Einschlagwerkzeug die äussere Kontur des übrigen Nagels nicht verlassen wird, daher keine überstehenden Teile vorhanden sind, die unnötig den Raum vergrössern und das Gewebe beeinträchtigen.

In Fig. 4 ist ein Knochennagel 30 im Querschnitt dargestellt, der annähernd rechteckförmig ist mit ebenfalls stark abgerundeten Kanten 31. Die Krümmungsebene fällt mit der grossen Achse 32 des Rechteckquerschnitts zusammen. Der Knochennagel 30 hat ein vergleichbares Widerstandsmoment wie ein Knochennagel von Kreisquerschnitt, wie er durch den gestrichelten Kreis 33 angedeutet ist. Ersterer hat daher eine erhebliche Materialeinsparung zur Folge, ohne dass die Tragfähigkeit dadurch geringer wäre.

Fig. 5 zeigt einen Nagel 35, der im Querschnitt annähernd die Form eines Malteserkreuzes hat, wobei die Kanten wiederum stark abgerundet sind, wie bei 36 dargestellt. Auch hier zeigt ein gestrichelter Kreis 37 einen Kreisquerschnitt eines Knochennagels, dessen Widerstandsmoment vergleichbar ist gegenüber dem des Knochennagels 35.

In Fig. 6 ist der Knochennagel 11 mit dem proximalen Endabschnitt dargestellt. Man erkennt einen im Durchmesser erweiterten Wulst 16, dessen Aussenkontur annähernd kreisförmig ist. Ferner ist der Wulst am freien Ende abgeflacht, wie bei 40 dargestellt. Die Abflachung liegt in einer Ebene, die annähernd senkrecht zur Belastungsrichtung ist, die durch einen Pfeil 41 angedeutet ist. Dieser Pfeil 41 ist auch in Fig. 1 gezeigt.

Es versteht sich, dass zusätzlich der Abschnitt 23 mit einer Bohrung bzw. einer Durchbohrung 23' versehen werden kann, um den Kraftangriff mit einem Einschlagwerkzeug zu verbessern. Eine Durchbohrung am distalen Ende kann im übrigen bei beliebigem Querschnitt geformt werden, um Einschlag- und Extraktionswerkzeug angreifen zu lassen.

Mit 23'' in Fig. 2 ist eine Bohrung angedeutet, die ausserhalb des ausgenommenen Abschnitts 21 dem Ende zugewandt angeordnet ist. Mit 23'''

in Fig. 3 ist eine dazu quer angeordnete Bohrung bezeichnet.

Der Nagel weist im übrigen am distalen Endabschnitt einen geraden Abschnitt auf, der durch einen grossen Bogen mit dem übrigen Nagel verbunden ist.

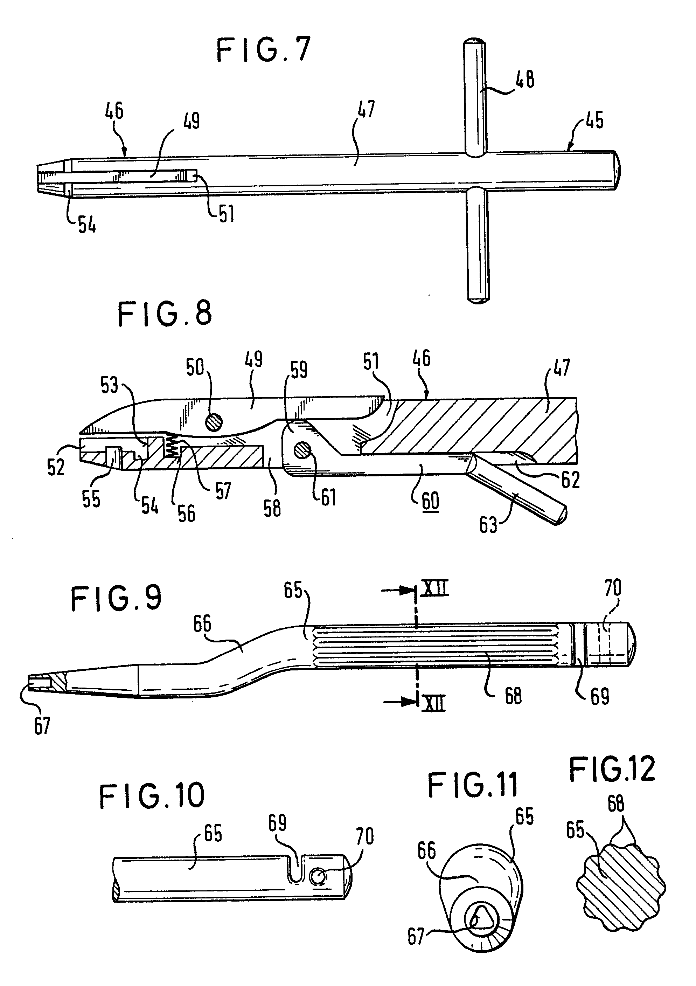

In den Fig. 7 und 8 ist ein Vorschlaggerät für in den Fig. 1 bis 6 dargestellte Knochennägel gezeigt. Es besteht aus einem Griffabschnitt 45 und einem Kupplungsabschnitt 46. Beide weisen einen langgestreckten im Querschnitt kreisförmigen Schaft 47 auf, der im Griffabschnitt 45 einen Querstab 48 aufweist, der durch eine entsprechende Bohrung hindurchgeführt ist. Im Kupplungsabschnitt 46 ist ein Kupplungshebel 49 schwenkbar gelagert (Fig. 8), und zwar mit Hilfe eines Lagerstiftes 50. Zwecks Aufnahme des Kupplungshebels 49 ist axial in den Schaft 47 ein Schlitz 51 eingeformt, der den relativ schmalen Kupplungshebel 49 aufnimmt, so dass er nur ein wenig über die Aussenkontur des Schaftes 47 übersteht. Der Kupplungshebel 49 erstreckt sich bis zum Ende des Schaftes 47 und überspannt eine im Querschnitt dreieckförmige Ausnehmung 52, die zum Ende des Schaftes 47 geöffnet ist und die an dem gegenüberliegenden Ende durch eine Anschlagwand 53 begrenzt ist, an deren Fuss sich eine Quernut 54 befindet. Ein im Schaft 47 eingelassener Zapfen 55 steht nach oben in die Ausnehmung 52.

Im Boden des Schlitzes 51 befindet sich eine Blindbohrung 56, die eine Druckfeder 57 aufnimmt, welche den Kupplungshebel 49 in Uhrzeigerrichtung beaufschlagt. Der Schlitz 51 ist nach unten in diametraler Richtung mit einer Durchbrechung 58 versehen zur Aufnahme eines Nockens 59 eines Klemmhebels 60. Der Klemmhebel 60 ist im Bereich des Nockens 59 mit Hilfe eines Lagerzapfens 61 schwenkbar gelagert, wobei die Hebel 49 und 60 annähernd in gleicher Ebene schwenkbar sind. Ein mittlerer Abschnitt des Hebels 60 ist in einer Ausnehmung 62 des Schaftes 47 teilweise aufgenommen. Eine Handhabe 63 erstreckt sich schräg nach aussen.

In der in Fig. 8 dargestellten Position ist der Kupplungshebel 49 durch den Klemmhebel 60 verriegelt. Der höchste Punkt des Nockens 59 liegt gegenüber der Drehachse des Zapfens 61 zum Schwenkzapfen 50 hin versetzt. Die Feder 57 versucht den Kupplungshebel 49 in Uhrzeigerrichtung zu drehen. Dadurch drückt der in Fig. 8 rechte Teil des Hebels 49 auf den Nocken 59 von oben und beaufschlagt den Klemmhebel 60 entgegengesetzt der Uhrzeigerrichtung. Eine Verschwenkung des Klemmhebels 60 in dieser Richtung ist jedoch nicht möglich, da sie durch den Schaft 57 bzw. die Ausnehmung 62 begrenzt wird. Erst wenn von Hand die Handhabe 63 erfasst und der Klemmhebel 60 ebenfalls in Uhrzeigerrichtung verdreht wird, kann der Kupplungshebel 59 ebenfalls im Uhrzeigersinn schwenken. Dadurch kann das distale Ende eines Knochennagels in die Ausnehmung 42 eingesetzt werden, wobei der Zapfen 55 z.B. in die Ausnehmung 21 eines Knochennagels nach den Fig. 1 bis 3 eingreift. Ein derart aufgenommener Knochennagel kann nun mit Hilfe des Vorschlagwerkzeugs nach den Fig. 7 und 8 eingeschlagen und in gewünschter Weise manipuliert werden, z.B. auch wieder ganz oder teilweise herausgezogen werden. Ein unabsichtliches Lösen des Geräts vom Knochennagel wird durch die beschriebene Klemmvorrichtung vermieden.

Da es vor allem bei mehreren Nägeln schwierig ist, mit Hilfe eines Vorschlaggeräts nach den Fig. 7 und 8 den Knochennagel bis zur Gänze einzutreiben, ist ein Nachschlaggerät nach den Fig. 9 bis 12 vorgesehen. Das Nachschlaggerät besitzt einen länglichen Schaft 65 von annähernd kreisförmigem Querschnitt, der im Bereich des vorderen Drittels bei 66 etwas gekröpft ist. Der Schaft 66 verjüngt sich zum vorderen Ende stetig. An seinem vorderen Ende weist er eine nach vorn geöffnete Ausnehmung 67 auf, deren Querschnitt dem Querschnitt des distalen Endes angepasst ist, also z.B. dreieckförmig, wie in Fig. 11 dargestellt. Der eigentliche Griffabschnitt weist eine Längsriffelung 68 auf, damit das Nachschlaggerät von Hand besser erfasst werden kann. Im hinteren Bereich ist in dem Schaft 65 ein nach aussen offener Querschlitz 69 eingeformt. Ferner ist parallel dazu eine Durchbohrung 70 vorgesehen (Fig. 10). Der Schlitz 69 hat eine Breite bzw. die Durchbohrung 70 hat einen Durchmesser, welche etwas grösser sind als der grösste Durchmesser des Knochennagels. Das Gerät nach den Fig. 9 bis 12 kann daher auch als sog. Schränkeisen dienen, mit dessen Hilfe es möglich ist, den Knochennagel in begrenzter Weise zu verformen, d.h. z.B. eine andere Krümmung oder Richtung zu geben. Vorzugsweise werden zwei derartige Geräte dabei eingesetzt.

Die Spitze des Nachschlaggerätes kann an der Unterseite eine Anfräsung oder Abflachung aufweisen, damit sie zu Ausschlagzwecken in die Nut 21 des Nagels 11 eingreifen kann. Auf diese Weise kann der implantierte Nagel so weit ausgeschlagen werden, bis das Vorschlaggerät eingesetzt werden kann.

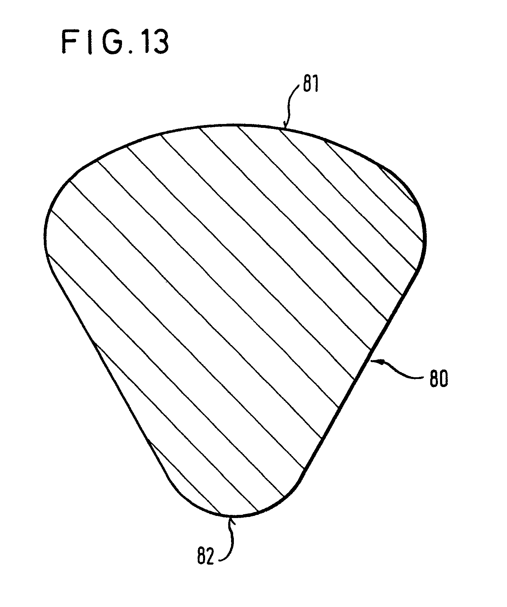

Das Profil des Nagels 80 nach Fig. 13 ist zwar ebenfalls grundsätzlich dreieckig wie das nach den Figuren 1 bis 3. Die konvexe Seite 81 ist jedoch nicht gerade, sondern im Querschnitt ballig. Der relativ grosse Radius der Seite 81 ergibt einen weichen Übergang zu den Radien an den Kanten des Nagels 80. Auch die konkav verlaufende Kante 82 ist mit einem Radius versehen.

**Patentansprüche**

1. Dünner flexibler Femurnagel zur Versorgung von Frakturen des proximalen Femurs durch Eintreiben eines oder mehrerer Nägel vom lateralen Femurkondylus in den Femurkanal bis in den Femurkopf, dessen distaler Abschnitt einen Kupplungsabschnitt für den Eingriff mit einem Einschlagwerkzeug aufweist und dessen zwischen dem distalen und proximalen Abschnitt liegender mittlerer Abschnitt bogenförmig gekrümmt ist, dadurch gekennzeichnet, dass der proximale Endabschnitt am freien Ende eine wulstartige, abgerundete Verdickung (16) aufweist, die ihrerseits am freien Ende eine Abflachung (40) aufweist, deren Ebene zur Aufnahme der Belastung im Femurkopf (16) im Winkel zur Achse des proximalen Nagelabschnitts verläuft.

2. Femurnagel nach Anspruch 1, dadurch gekennzeichnet, dass der Querschnitt des Nagels (11) über seine Länge polygonal ist mit abgerundeten Kanten.

3. Femurnagel nach Anspruch 2, dadurch gekennzeichnet, dass der Querschnitt dreieckig ist, vorzugsweise ein gleichseitiges Dreieck bildet.

4. Femurnagel nach Anspruch 3, dadurch gekennzeichnet, dass die konvexe Seite des Nagels (80) im Querschnitt ballig oder kreisbogenförmig gewölbt ist.

5. Femurnagel nach Anspruch 2, dadurch gekennzeichnet, dass der Querschnitt rechteckig ist und die grössere Achse (32) in der Krümmungsebene liegt.

6. Femurnagel nach Anspruch 2, dadurch gekennzeichnet, dass der Querschnitt sternförmig ist.

7. Femurnagel nach einem der Ansprüche 1 bis 6, dadurch gekennzeichnet, dass im distalen Ende eine Bohrung vorgesehen ist, vorzugsweise eine Durchbohrung (70), deren Achse vorzugsweise in der Krümmungsebene liegt.

8. Femurnagel nach einem der Ansprüche 1 bis 7, dadurch gekennzeichnet, dass im Abstand vom distalen Ende eine Ausnehmung (21) geformt ist zur Bildung eines im Querschnitt polygonalen Einspannabschnitts (23).

9. Femurnagel nach Anspruch 3 und 9, dadurch gekennzeichnet, dass die Ausnehmung (21) durch Materialwegnahme von einer Eckkante (19) ausgehend geformt ist.

10. Femurnagel nach Anspruch 9, dadurch gekennzeichnet, dass der Boden (22) der Ausnehmung (21) annähernd parallel zu der gegenüberliegenden Seite (18) verläuft.

## Claims

1. Thin flexible femur nail for the treatment of fractures of the proximal femur part by driving one or a plurality of nails from the lateral femur condylus into the femur tube up to the femur head whose distal portion has a coupling portion for engagement with a hammer and whose central portion lying between the distal and proximal portions is arcuate, characterized in that the proximal end portion has at the free end a bead-like rounded-off thickness (16) which in turn has at the free end an oblateness (40) whose plane for receiving the loading on the femur head (16) extends at an angle to the axis of the proximal nail portion.

2. Femur nail as in claim 1, characterized in that the cross-section of the nail (11) is polygonal over its length with rounded-off edges.

3. Femur nail as in claim 2, characterized in that the cross-section is triangular, preferably defining an equilateral triangle.

4. Femur nail as in claim 3, characterized in that the convex side of the nail (80) is crowned or circular-arc-shaped.

5. Femur nail as in claim 2, characterized in that the cross-section is rectangular, with the greater axis (32) lying in the plane of curvature.

6. Femur nail as in claim 2, characterized in that the cross-section is stellate.

7. Femur nail as in any of claims 1 to 6, characterized in that the distal end has provided therein a bore, preferably a through-bore (70) whose axis lies preferably in the plane of curvature.

8. Femur nail as in any of claims 1 to 7, characterized in that a recess (21) is formed at a distance from the distal end to provide a clamping portion (23) polygonal in cross-section.

9. Femur nail as in claims 3 and 9, characterized in that the recess (21) is formed by relieving from a corner edge (19).

10. Femur nail as in claim 9, characterized in that the bottom (22) of the recess (21) extends approximately in parallel to the opposite side (18).

## Revendications

1. Clou fémoral flexible mince pour le traitement de fractures du fémur proximal par enfoncement à partir du condyle fémoral latéral dans le canal fémoral jusque dans la tête du fémur d'un ou plusieurs clous, dont le tronçon distal présente un tronçon d'accouplement pour l'intervention avec un outil d'enfoncement et dont le tronçon central, situé entre les tronçons distal et proximal, est courbé en forme d'arc, caractérisé en ce que le tronçon terminal proximal présente à l'extrémité libre un épaississement arrondi (16) similaire à un bourrelet, qui présente à son tour à l'extrémité libre un plat (40) dont le plan, pour absorber la charge dans la tête du fémur (16), est dirigé sous un angle par rapport à l'axe du tronçon proximal de clou.

2. Clou fémoral selon la revendication 1, caractérisé en ce que la section du clou (11) est polygonale sur sa longueur, avec arêtes arrondies.

3. Clou fémoral selon la revendication 2, caractérisé en ce que la section est triangulaire, et de préférence forme un triangle équilatéral.

4. Clou fémoral selon la revendication 3, caractérisé en ce que le côté convexe du clou (80) est, en section, bombé sphériquement ou en forme d'arc de cercle.

5. Clou fémoral selon la revendication 2, caractérisé en ce que la section est rectangulaire et que le grand axe (32) est situé dans le plan de courbure.

6. Clou fémoral selon la revendication 2, caractérisé en ce que la section est en forme d'étoile.

7. Clou fémoral selon l'une des revendications 1 à 6, caractérisé en ce qu'un trou, de préférence un trou traversant (70), dont l'axe est de préférence situé dans le plan de courbure, est prévu dans l'extrémité distale.

8. Clou fémoral selon l'une des revendications 1 à 7, caractérisé en ce qu'un évidement (21) pour la formation d'un tronçon de serrage à section (23) polygonale est formé à une certaine distance de l'extrémité distale.

9. Clou fémoral selon les revendications 3 et 8, caractérisé en ce que l'évidement (21) est formé par enlèvement de matière en partant d'une arête d'angle (19).

10. Clou fémoral selon la revendication 9, caractérisé en ce que le fond (22) de l'évidement (21) est dirigé approximativement parallèlement au côté opposé (18).

# FIG.1

FIG.2

FIG.3

FIG.4

FIG.5

FIG.6

FIG.7

FIG.8

FIG.9

FIG.10

FIG.11

FIG.12

# FIG.13